# EUROPÄISCHE PATENTANMELDUNG

(11) **EP 3 095 522 A1**
(43) Veröffentlichungstag der Anmeldung: **23.11.2016**
(21) Anmeldenummer: 15168316.6
(22) Anmeldetag: 20.05.2015
(51) Int. Cl.: B05B 1/14, B65D 83/20, B65D 83/30, B65D 83/14, A61M 15/00, A61M 11/00, B65D 83/24

(54) **INHALATIONSEINRICHTUNG, INHALATIONSEINRICHTUNGS-SET UND DÜSENPLATTE HIERFÜR**

(71) Anmelder: Aptar Radolfzell GmbH, 78315 Radolfzell (DE)
(72) Erfinder: Nadler, Günter, 78345 Moos-Iznang (DE)
(74) Vertreter: Patentanwaltskanzlei Cartagena

(57) **Zusammenfassung**

Bekannt ist eine Inhalationseinrichtung (10) zum Inhalieren einer Flüssigkeit in vernebelter Form. Die Inhalationseinrichtung (10) verfügt über ein Gehäuse (20), welches einen Flüssigkeitsspeicher (21) umgibt, in dem die Flüssigkeit vor dem Austrag gelagert ist, und über einen Applikatorkopf (40) mit einem Zerstäubungsraum (42) und einem hiermit verbundenen Applikatorstück (80; 86), wobei das Applikatorstück (80; 86) entweder als Mundstück (86) zur Aufnahme im Mund eines Patienten oder als Inhalationsmaske (80) zur dichten Überdeckung des Mundes, der Nase oder des Mundes und der Nase ausgebildet ist. Die Inhalationseinrichtung (10) verfügt weiterhin über einen Austragkanal (26), der den Flüssigkeitsspeicher (21) mit dem Applikatorkopf (40) verbindet.

Es wird vorgeschlagen, dass die Inhalationseinrichtung (10) am Ende des Austragkanals (26) über eine Düsenplatte (90) mit einer Vielzahl von Düsenöffnungen (92) zur Erzeugung eines Inhalationsnebels verfügt, durch die hindurch die Flüssigkeit vom Flüssigkeitsspeicher (21) in den Zerstäubungsraum (42) geleitet wird.

Verwendung insbesondere zum Austrag von Salzlösungen in vernebelter Form.

## Beschreibung

Die Erfindung betrifft eine Inhalationseinrichtung zum Inhalieren einer Flüssigkeit in vernebelter Form nach dem Oberbegriff von Anspruch 1. Die Erfindung betrifft weiterhin ein Inhalationseinrichtungs-Set nach dem Oberbegriff von Anspruch 11 und eine Düsenplatte nach dem Oberbegriff von Anspruch 11.

Inhalationseinrichtungen zum Inhalieren einer Flüssigkeit in vernebelter Form sind bekannt.

Gattungsgemäße Inhalationseinrichtungen dienen dem Zweck, Flüssigkeit zu vernebeln, so dass dieses durch Einatmen in die Bronchien eines Patienten gelangen kann. Neben klassischen Medikamenten finden solche Inhalationseinrichtungen insbesondere auch Anwendung zum Austrag von Salzwasser oder anderweitig mit ätherischen Ölen ergänztem Wasser. Hierdurch wird an Erkältung und Grippe Erkrankten eine einfache Möglichkeit gegeben, die Atmung wieder zu erleichtern und Schmerzen im Nasen-, Hals- und Rachenraum sowie in der Lunge mit einfachen Mitteln zu lindern.

Bekannte Inhalationseinrichtungen sind meist entweder sehr einfach aufgebaut, indem sie nicht sehr viel mehr als eine mit einem Speicher verbundene Atemmaske zur Verfügung stellen, wobei der Speicher für jede Anwendung neu zu befüllen ist, oder sie sind eher komplex aufgebaut sind und zum Teil mit einer Stromversorgung versehen sind, um die Vernebelung des Fluids zu gestatten. Solche letztgenannten Vorrichtungen sind häufig nicht ohne weiteres transportabel.

### AUFGABE UND LÖSUNG

Aufgabe der Erfindung ist es, eine Inhalationseinrichtung zur Verfügung zu stellen, die gegenüber den bekannten Inhalationseinrichtungen in Hinblick auf Transportabilität, einen einfachen Aufbau, eine bequeme Bedienung und/oder geringe Herstellungskosten von Vorteil ist.

Aufgabe der Erfindung ist es weiterhin, ein hierfür geeignetes Vernebelungsmittel zur Verfügung zu stellen sowie weitere Anwendungsfelder für dieses Vernebelungsmittel zu benennen.

Die Aufgabe wird durch eine Inhalationseinrichtung zum Inhalieren einer Flüssigkeit in vernebelter Form nach Anspruch 1 gelöst.

Die Inhalationseinrichtung verfügt über ein Gehäuse, welches einen Flüssigkeitsspeicher umgibt, in dem die Flüssigkeit vor dem Austrag gelagert ist. Die Inhalationseinrichtung verfügt über einen Applikatorkopf mit einem Zerstäubungsraum und einem hiermit verbundenen Applikatorstück, wobei das Applikatorstück entweder als Mundstück zur Aufnahme im Mund eines Patienten oder als Inhalationsmaske zur dichten Überdeckung des Mundes, der Nase oder des Mundes und der Nase oder als Adapterstück zur Anbringung eines Mundstücks oder einer Inhalationsmaske ausgebildet ist. Die Inhalationseinrichtung verfügt über einen Austragkanal, der den Flüssigkeitsspeicher mit dem Applikatorkopf verbindet.

Die Inhalationseinrichtung verfügt am Ende des Austragkanals über eine Düsenplatte mit einer Vielzahl von Düsenöffnungen zur Erzeugung eines Inhalationsnebels, durch die hindurch die Flüssigkeit vom Flüssigkeitsspeicher in den Zerstäubungsraum geleitet wird.

Eine erfindungsgemäße Inhalationseinrichtung verfügt über eine speziell an den Inhalationszweck angepasste Form eines Applikatorstücks.

Dieses kann als Mundstück zur rein oralen Aufnahme des Inhalationsnebels vorgesehen sein. Als Mundstück weist das Applikatorstück üblicherweise einen Auslass auf, dessen Breite größer ist als seine Höhe und der an der Oberseite und an der Unterseite über Anlageflächen zum Anlegen der Oberlippe und der Unterlippe verfügt. Durch die gegenüberliegenden Anlageflächen kann das Mundstück von den Lippen des Benutzers fest umgriffen werden, was bei nachfolgend noch beschriebenen Ventilkonstellationen von Vorteil sein kann.

Als Inhalationsmaske weist das Applikatorstück üblicherweise eine umlaufende Dichtkante zur Anlage am Gesicht eines Benutzers auf, wobei die Dichtkante so bemessen ist, dass sie den Mund oder die Nase oder den Mund und die Nase umschließen kann. Die Inhalationsmaske kann mit Haltemitteln wie beispielsweise einem Gummizug versehen sein, welches es gestattet, die Maske fest vor Mund und/oder Nase zu positionieren, ohne sie hier gezielt halten zu müssen.

Das unmittelbar am Applikatorkopf angebrachte Applikatorstück kann auch ein Adapterstück sein, welches zu Ankoppelung eines Mundstücks oder einer Inhalationsmaske ausgebildet sein kann. Dass Mundstück und/oder die Inhalationsmaske können dann als separate Teile der Inhalationseinrichtung für die Montage durch den Endverbraucher beigefügt sein. Insbesondere kann eine Steckverbindung vorgesehen sein, wobei am Mundstück oder der Inhalationsmaske ein Steckabschnitt vorgesehen ist, der in das Applikatorstück eingeschoben oder auf dieses aufgeschoben wird. Der Steckabschnitt und das Applikatorstück gehen dann vorzugsweise eine kraftschlüssige Verbindung ein.

Auch ist es möglich, dass das Applikatorstück selbst bereits als Mundstück verwendbar ist, dennoch aber das Einstecken oder Aufstecken eines Steckabschnitts einer mitgelieferten Inhalationsmaske gestattet.

Ein Auslass des Applikatorstücks in Art eines Adapterstücks oder als Mundstück weist vorzugsweise einen minimalen freien Querschnitt zwischen 50 mm² und 2000 mm² auf, vorzugsweise zwischen 80 mm² und 800 mm², insbesondere zwischen 500 mm² und 800 mm².

Die Erzeugung von Inhalationsnebel wird bei einer erfindungsgemäßen Inhalationseinrichtung über eine Düsenplatte mit einer Vielzahl kleiner Düsen erzielt. Eine solche Düsenplatte weist eine Mehrzahl von Düsenöffnungen auf, wobei vorzugsweise 9 oder mehr Düsenöffnungen, insbesondere vorzugsweise 16 oder mehr Düsenöffnungen oder 25 oder mehr Düsenöffnungen vorgesehen sind. Diese verfügen vorzugsweise über einen Durchmesser zwischen 1 µm und 100 µm, insbesondere zwischen 2 µm und 10 µm.

Eine solche Düsenplatte hat sich als besonders einfache Möglichkeit herausgestellt, um einen gut inhalierbaren Inhalationsnebel insbesondere aus Salzwasser zu erzeugen. Die Düsenplatte wird während des Austrags konstant mit Flüssigkeit beströmt, die durch die Düsenöffnungen hindurchgedrückt und dadurch vernebelt wird.

Der entstandene Inhalationsnebel gelangt in den Zerstäubungsraum innerhalb des Applikatorkopfes, der mit dem Applikationsstück verbunden ist. Durch Einatmen bei angesetztem Applikationsstück kann dieser Inhalationsnebel inhaliert werden.

Die Flüssigkeit im Flüssigkeitsspeicher kann durch Beaufschlagung mit Treibgas, mit Druckluft oder durch eine vorgespannte Federeinrichtung unter Druck steht.

Obwohl grundsätzlich auch möglich ist, die Flüssigkeit zur Erzeugung des Inhalationsnebels mittels einer manuellen Pumpeinrichtung herzustellen, ist es von Vorteil, dass die Inhalationseinrichtung bereits über einen solchen Energiespeicher mechanischer oder chemischer Art verfügt, der zu einem gleichbleibenden Flüssigkeitsdruck und damit zu einem gleichbleibenden Zerstäubungsergebnis führt. Neben der hierfür vorgesehenen Möglichkeit, im Flüssigkeitsspeicher Treibgas zu lagern, wird auch die Möglichkeit als vorteilhaft angesehen, einen separaten

Überdruck-Luftspeicher zu haben, dessen Druck auf den Flüssigkeitsspeicher wirkt. Auch eine während der Fertigung der Inhalationseinrichtung bzw. des Flüssigkeitsspeichers vorgespannte Feder, die über einen Kolben permanent die Flüssigkeit druckbeaufschlagt, ist eine vorteilhafte und insbesondere auch umweltfreundliche Möglichkeit der Energiespeicherung.

Der Flüssigkeitsspeicher selbst kann ein definiertes Volumen aufweisen, so dass nach dem Austrag zum Ausgleich Luft hierein einströmt. Es sind aber auch unbelüftete Systeme möglich, die über einen Schleppkolben oder einen Beutel ein veränderliches Volumen des Flüssigkeitsspeichers gestatten.

Die Inhalationseinrichtung kann über ein schaltbares Dreh-Auslassventil verfügen, durch das der Austragkanal geöffnet und geschlossen werden kann. Das Dreh-Auslassventil kann durch eine Drehbewegung des Gehäuses oder eines am Gehäuse oder am Applikatorkopf vorgesehenen Schaltelements gegenüber dem Applikatorstück schaltbar sein. Das Ventil ist der Düsenplatte vorgeschaltet.

Das Gehäuse kann zumindest abschnittsweise als rotationssymmetrischer Körper ausgestaltet sein, dessen Mittelachse eine Haupterstreckungsachse des Gehäuses definiert. Das Gehäuse oder das Schaltelement kann um diese Haupterstreckungsachse gegenüber dem Applikatorstück drehbar sein.

Das Vorsehen eines schaltbaren Auslassventils gestattet es, hierüber den Austrag von Inhalationsnebel zu steuern. Das genannte Dreh-Auslassventil ist ein Ventil, welches durch die genannten Möglichkeiten einer Relativdrehbewegung geöffnet und geschlossen werden kann, wobei als Dreh-Auslassventil auch ein rein axial wirkendes Ventil verstanden wird, wenn dieses mit einer Ventilmechanik versehen wird, durch die eine rotative Relativbewegung zu einer solchen axialen Bewegung umgewandelt wird.

Die Drehbewegung zum Zwecke des Öffnens und Schließens des Auslassventils hat sich als sehr einfache und insbesondere gut einhändig realisierbare Möglichkeit herausgestellt. Wenn das Applikatorstück im Mundbereich oder im Mund- und Nasenbereich des Benutzers fixiert wird oder auch nur als Maske gegen das Gesicht des Benutzers gedrückt wird, kann auf einfache Weise mit einer Hand das Gehäuse zum Zwecke des Öffnens oder Schließens gedreht werden. Auch ein Schaltring oder dergleichen kann gut mit jener Hand bedient werden, die das Gehäuse umgreift.

Die Inhalationseinrichtung kann über ein schaltbares Schwenk-Auslassventil verfügen, durch das der Austragkanal geöffnet und geschlossen werden kann. Das Schwenk-Auslassventil kann durch eine Schwenkbewegung des Applikatorstücks gegenüber dem Gehäuse schaltbar sein.

Das Gehäuse kann zumindest abschnittsweise als rotationssymmetrischer Körper ausgestaltet sein, dessen Mittelachse eine Haupterstreckungsachse des Gehäuses definiert. Das Applikatorstück kann um eine zu dieser Haupterstreckungsachse orthogonale Schwenkachse gegenüber dem Gehäuse schwenkbar sein.

Als Schwenkauslassventil wird ein Ventil verstanden, welches durch eine Schwenkbewegung zwischen Applikatorstück und Gehäuse schaltbar ist. Auch bei einem solchen Ventil kann es sich um ein Ventil handeln, dessen Ventilkörper und Ventilsitz lediglich axial gegeneinander bewegt werden, sofern eine Ventilmechanik vorgesehen ist, die die Schwenkbewegung in eine solche axiale Bewegung überführt.

Auch die Schwenkbewegung, insbesondere um eine zur Haupterstreckungsachse des Gehäuses orthogonale Schwenkachse, hat sich als sehr vorteilhafte Möglichkeit zur Steuerung der Inhalationseinrichtung herausgestellt. Während eine rein axiale Bewegung, insbesondere bei angesetztem Applikatorstück, häufig schwer fällt und insbesondere schwer dosierbar ist, ist die Schwenkverstellung des Gehäuses, welches während des Inhalationsvorgangs ohnehin umgriffen wird, motorisch einfach und dosiert möglich.

Zur Erzielung der Schwenkbewegung oder anderer zur Ventilöffnung vorgesehener Relativbewegungen kann eine gegenüber dem Gehäuse bewegliche Betätigungshandhabe vorgesehen sein.

Dem Auslassventil kann eine Federeinrichtung zugeordnet sein, die derart ausgebildet ist, dass ihre Federkraft stets versucht, das Auslassventil zu schließen.

Eine solche Federeinrichtung kann unmittelbar Teil des Auslassventils sein. Die Federeinrichtung kann jedoch auch zwischen dem Gehäuse und dem Applikatorkopf bzw. dem Gehäuse und dem Applikatorstück vorgesehen sein und nur mittelbar auf das Ventil wirken.

Die Inhalationseinrichtung kann verfügt über ein druckabhängig schaltbares Auslassventil verfügen, durch das der Austragkanal geöffnet und geschlossen werden kann. Das druckabhängig schaltbare Auslassventil kann als atembetätigtes Auslassventil ausgestaltet sein, welches durch Erzeugung eines Unterdrucks am Applikatorstück geöffnet werden kann und welches durch Erzeugung eines Überdrucks am Applikatorstücks oder durch Wegfall des Unterdrucks geschlossen werden kann.

Ein druckabhängig schaltbares Auslassventil bietet den Vorteil, dass der Benutzer jederzeit die Inhalation unterbrechen kann, sei es für einen längeren Zeitraum von beispielsweise einigen Minuten oder nur für einen kurzen Augenblick, um auszuatmen. Wenn der durch den Benutzer erzeugte Unterdruck entfällt, schließt das Auslassventil, so dass keine Flüssigkeit vergeudet wird.

Die druckabhängig schaltbare Auslassventilanordnung ist dabei mechanisch ausgebildet. Dies bedeutet, dass der Unterdruck selbst die Verlagerung eines Bauteils bewirkt, welches mittelbar das Ventil öffnet oder schließt. Insbesondere von Vorteil ist es, wenn ein am Applikatorkopf vorgesehener Einlasskanal, durch den während der Inhalation Luft in die Zerstäubungskammer eingesogen wird, mit einer Klappe oder dergleichen versehen ist, die bei Unterdruck ausgelenkt wird und dadurch das Ventil öffnet.

Die Inhalationseinrichtung kann über eine bistabile schaltbare Auslassventileinrichtung verfügen.

Eine bistabil schaltbare Auslassventileinrichtung gestattet es, neben einer Deaktivierung auch eine dauerhafte Aktivierung des Zerstäubungsvorganges zu bewirken. Der jeweilige Ventilzustand muss nicht durch dauerhafte Einbringung einer externen Kraft aufrechterhalten werden.

Die bistabile schaltbare Auslassventileinrichtung kann mit einer Ventil mechanik versehen sein, durch die Kraftbeaufschlagungen in gleicher Richtung alternierend das Öffnen und das Schließen der Auslassventileinrichtung verursachen.

Eine solche Mechanik wird häufig auch als Push/Push-Mechanik, Herzkurvenmechanik oder Kugelschreibermechanik bezeichnet. Gleichgerichtete Kraftbeaufschlagungen einer Steuerfläche können hier alternierend das Öffnen und Schließen des Ventils bewirken.

Die Inhalationseinrichtung kann über eine Signalisierungseinrichtung verfügen, die bei erfolgender Inhalation am Applikatorstück und durch den hierdurch erzeugen Unterdruck aktiviert wird. Eine solche Signalisierungseinrichtung gestattet es erwachsenen Benutzern, zu erkennen, ob sie die Inhalationseinrichtung in richtiger Art und Weise verwenden. Insbesondere hilfreich ist eine Signalisierungseinrichtung, wenn die Inhalationseinrichtung durch Kinder verwendet wird und die Eltern eine Möglichkeit wünschen, zu erkennen, ob das Kind in richtiger Art und Weise inhaliert.

Die Signalisierungseinrichtung kann als Tonerzeuger ausgebildet sein, insbesondere als Pfeife oder als Tonerzeuger mit einem durch einen Luftstrom rotierenden Tongeber. Die Signalisierungseinrichtung kann auch als optische Signalisierungseinrichtung ausgebildet sein und einen von außen sichtbaren Signalabschnitt aufweisen, der durch Unterdruck beim Inhalieren aus einer nicht signalisierenden Lage in eine Signalisierungslage gedrückt wird oder in Bewegung versetzt wird.

Eine als Tonerzeuger ausgebildete Signalisierungseinrichtung zeichnet sich dadurch aus, dass sie akustisch verdeutlicht, ob die Betätigung auf richtige Art und Weise erfolgt. Hier bieten sich insbesondere pfeifenähnliche Gestaltungen an, wobei durch die Formgebung dessen gesteuert werden kann, bei welchem Unterdruck ein die richtige Verwendung kennzeichnender Ton erzeugt werden soll. Auch unterschiedliche Töne oder Tonhöhen in Abhängigkeit der Inhalationsleistung sind denkbar.

Als optische Signalisierungseinrichtung weist die Signalisierungseinrichtung einen Signalabschnitt auf, der verlagert oder bewegt wird, um die richtige Art der Inhalation zu kennzeichnen. Auch hier kommt eine trillerpfeifenähnliche Gestaltung in Betracht, bei der ein Körper durch den Luftstrom entlang eines gleichbleibenden umlaufenden Pfades bewegt wird. Dieser Körper muss von außen ersichtlich sein, um ihn bestimmungsgemäß nutzen zu können, beispielsweise durch eine transparente Wandung hindurch. Eine Alternative sieht vor, dass ein Signalabschnitt in Abhängigkeit der Stärke des Unterdrucks am Applikatorstück in verschiedenem Maße verlagert wird. So kann beispielsweise ein Pfeil auf einer Skala kennzeichnen, wie groß der vom Benutzer erzeugte Unterdruck ist. Auch ein kleines rotierendes Schaufelrad kann als Signalisierungsabschnitt Verwendung finden.

Der Applikatorkopf kann über ein drehfest mit dem Gehäuse verbundenes Basisteil und über ein demgegenüber drehbares Auslassteil, welches den Zerstäubungsraum umfasst, verfügen. Der Applikatorkopf und das Auslassteil aneinander derart geführt sein, dass eine Drehbewegung des Auslassteils gegenüber dem Basisteil eine axiale Verlagerung der Auslassteils gegenüber dem Basisteil bewirkt, durch die ein Auslassventil der Inhalationseinrichtung schaltbar ist.

Dies gestattet eine besonders einfache Bauweise des Applikatorkopfes mit nur zwei relativbeweglichen Teilen und insgesamt mitsamt Düsenplatte nur drei Bauteilen. Die Basis wird am Gehäuse der Inhalationseinrichtung angebracht. Sie verfügt vorzugsweise über eine Führungsschräge, insbesondere als Teil eines Innengewindes oder einer Kulisse. Das zweite Teil, das Auslassteil, ist drehbar an der Basis angebracht, wobei es durch Führungsabschnitte an der Führungsschräge geführt ist, so dass die Drehbewegung auch eine axiale Verlagerung zur Folge hat. Diese axiale Verlagerung kann ein Ventil öffnen, welches im Gehäuse in der Nähe des Flüssigkeitsspeichers oder am Applikatorkopf vorgesehen sein kann. Das Auslassteil umfasst vorzugsweise sowohl die Zerstäubungskammer als auch einen darin mündenden Auslass mit Düsenplatte. Das Auslassteil kann daher sehr einfach als Ganzes unter dem Wasserhahn gereinigt werden.

Im Flüssigkeitsspeicher kann insbesondere eine wässrige Lösungen, vor allem eine salzhaltige wässrige Lösung enthalten. Weiterhin kann die Lösung eine gepufferte Lösung oder eine Ringerlösung darstellen. Es kann sich unmittelbar um Meerwasser handelt. Die wässrige Lösung kann mit verschiedenen Zusätzen versehen sein. Insbesondere können Kohlenhydrate, ätherische Öle, Menthol und Pflanzenextrakte enthalten sein. Auch enthalten sein können Vitamine und Spurenelemente sowie Mangan oder Zink. Auch Träger- oder Hilfsstoffe als Träger aktiver Substanzen können enthalten sein. Die Flüssigkeit im Flüssigkeitsspeicher kann insbesondere auch Zusätze aus der Gruppe umfassen Zimtöl, Teebaumöl, Salbeiöl, Thymianöl, Zitronenmelissenöl enthalten.

Der Austragkanal kann zumindest abschnittsweise mit einem antibakteriell wirkenden Material versehen sein, insbesondere mit Silber oder ein Silber beinhaltendes Material.

Die Verwendung eines antibakteriell wirkenden Materials im Austragkanal stromaufwärts der Düsenplatte hat sich als besonders vorteilhaft herausgestellt, da die Düsenplatte ein Trocknen des Austragkanals nach erfolgtem Austrag nur in geringem Maße gestattet. Die Verdunstung durch die dünnen Düsenöffnungen hindurch ist gering. Das antibakteriell wirkende Material kann entlang des Kanals an der Wandung aufgebracht sein. Denkbar ist auch eine poröse oder schwammartige Struktur aus antibakteriellem Material, die im Austragkanal angeordnet ist. Besonders bevorzugt wird es, wenn eine Innenseite der Düsenplatte antibakteriell ausgestaltet ist, beispielsweise durch eine Silberbeschichtung.

Das Applikatorstück und die Düsenplatte können Teil einer gemeinsamen Baueinheit sein, die werkzeuglos vom Gehäuse lösbar ist.

Die Vereinigung des Applikatorstücks und der Düsenplatte in einer gemeinsamen Baueinheit, die beispielsweise mittels Rastverbindungsmitteln einfach vom Gehäuse lösbar und wieder aufsetzbar ist, bietet den Vorteil separater Waschbarkeit einschließlich der Düsenplatte. Auch kann hierdurch in einfacher Weise ein Austausch dieser Baugruppe aus hygienischen Gründen erfolgen.

Die Aufgabe wird auch durch ein Inhalationseinrichtungs-Set nach Anspruch 11 gelöst.

Dieses Set umfasst eine Inhalationseinrichtung nach vorbeschriebener Art. Es umfasst mindestens zwei Baueinheiten, die jeweils ein Applikatorstück und eine Düsenplatte umfassen.

Ein solches Set gestattet es, ohne hygienische Bedenken die Inhalationseinrichtung durch mehrere Personen verwenden zu lassen, indem jede Person jeweils nur ihre eigene austauschbare Baueinheit verwendet. Da diese die größte Gefahr einer Kontamination birgt, wird Ansteckungsgefahr hierdurch wirksam vermieden.

Die Aufgabe wird auch durch eine Düsenplatte, insbesondere für eine Inhalationseinrichtung vorbeschriebener Art, nach Anspruch 12 gelöst.

Die Düsenplatte umfasst eine Vielzahl von Düsenöffnungen. Die Düsenplatte ist zumindest abschnittsweise aus Silber oder aus einem Silber beinhaltenden Material gebildet.

Es hat sich gezeigt, dass die Verwendung von Silber an einer Düsenplatte wirksam die Kontamination verhindert, insbesondere auch die Kontamination in den Düsenöffnungen selbst.

Die Düsenplatte kann Düsenöffnungen mit einem Durchmesser zwischen 1 µm und 100 µm, insbesondere zwischen 2 µm und 10 µm, umfassen. Die Düsenplatte kann mindestens 9 Düsenöffnungen, vorzugsweise mindestens 16 Düsenöffnungen und insbesondere vorzugsweise mindestens 25 Düsenöffnungen, umfassen.

Die Düsenplatte kann als Ganzes aus Silber oder einer Silberlegierung gefertigt sein. Dies wird jedoch aus wirtschaftlichen Erwägungen üblicherweise nicht als ideal angesehen.

Stattdessen kann die Düsenplatte eine Grundplatte aus einem Material aufweisen, welches kein Silber oder Silber beinhaltendes Material beinhaltet. Auf der Grundplatte kann eine Schicht aus Silber oder einem Silber beinhaltenden Material wie einer Silberlegierung vorgesehen sein.

Die Schicht aus Silber oder einem Silber beinhaltenden Material kann nach Einbringung der Düsenöffnungen durch Bedampfen oder Eintauchen auf die Grundplatte aufgebracht sein, so dass die Düsenöffnungen an ihren Innenseiten zumindest teilweise mit dieser Schicht bedeckt sind.

Die Grundplatte kann beispielsweise aus Silizium gefertigt sein, welches sich hierfür als sehr gut geeignetes Material herausgestellt hat. Das Silber oder das Silber enthaltende Material ist darauf in Form einer dünnen Schicht aufgebracht. Diese Schicht kann innenseitig und/oder außenseitig und/oder in den Düsenöffnungen selbst gegeben sein.

Die Düsenöffnungen können nach Bedampfen oder Eintauchen der Grundplatte zum Zwecke der Herstellung der Schicht aus Silber oder Silberoxid eingebracht sein, so dass die Düsenöffnungen an ihrer Innenseite zumindest teilweise frei von dieser Schicht sind.

Ein solches Vorgehen in der Fertigung kann wirtschaftlich erhebliche Vorteile bringen, da große plattenförmige Rohlinge bedampft werden können, aus denen erst später einzelne Düsenplatten erstellt werden. Bei ausreichend großem Düsendurchmesser ist die Gefahr der Verkeimung innerhalb der Düsen gering, zumindest dann, wenn innenseitig und/oder außenseitig die flächigen Bereiche der Düsenplatte mit einer entsprechenden Schicht versehen sind.

Die Aufgabe kann auch durch einen Spender zum Austragen von Flüssigkeiten in vernebelter Form, insbesondere ausgestaltet als Inhalationseinrichtung vorbeschriebener Art, gelöst werden.

Der Spender verfügt über ein Gehäuse, welches einen Flüssigkeitsspeicher umgibt, über einen Applikator und über einen Austragkanal, der den Flüssigkeitsspeicher mit dem Applikator verbindet.

Der Spender verfügt über eine Düsenplatte vorbeschriebener Art, durch die hindurch die Flüssigkeit am Applikator in eine Umgebung abgegeben wird.

Obwohl die Verwendung einer beschriebenen Düsenplatte insbesondere in einer Inhalationseinrichtung der beschriebenen Art als besonders vorteilhaft angesehen wird, könnten auch andere Spender eine solche antibakteriell ausgestaltete Düsenplatte verwenden. So sind beispielsweise Nasalspender zur nasalen Verabreichung eines Sprays ebenfalls mit solchen Düsenplatten denkbar.

Obwohl in der oben stehenden Beschreibung in Hinblick auf die Inhalationseinrichtung die Verwendung einer Düsenplatte als wesentliches Merkmal angesehen wird, ist es grundsätzlich auch denkbar, einige der genannten Aspekte ohne eine solche Düsenplatte zu realisieren.

Auch eine solche Gestaltung mit einer anderen Technik der Vernebelung kann einige der hier beschriebenen Aspekte vorteilhafterweise nutzen. So sind insbesondere die möglichen Ausgestaltungen eines Auslassventils oder der Aufteilung in Baugruppen gemäß den Ansprüchen 2 bis 6 und 8 sowie die Gestaltung mit Signalisierungseinrichtung gemäß Anspruch 7 separate Erfindungen, die nicht zwingend eine Düsenplatte zum Zwecke der Vernebelung benötigen.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der stark schematisierten Figuren erläutert sind.
Die Figuren 1a bis 1c zeigen verschiedene Ausgestaltungen von Düsenplatten, insbesondere für Inhalationseinrichtungen.
Fig. 2 sowie 3a und 3b zeigen besonders einfache Ausgestaltungen von Inhalationseinrichtungen.
Fig. 4, 4a und 4b zeigen eine Ausgestaltung einer Inhalationseinrichtung mit einem bistabilen Ventil mit einer kugelschreiberähnlichen Mechanik.
Fig. 5a und 5b sowie 6a und 6b zeigen eine Inhalationseinrichtung mit einem DrehAuslassventil.
Fig. 7a und 7b sowie Fig. 7c zeigen eine Inhalationseinrichtung mit einem SchwenkAuslassventil.
Fig. 8a und 8b zeigen eine Inhalationseinrichtung mit einem per Unterdruck gesteuerten Auslassventil.
Fig. 9a und 9b zeigen eine Inhalationseinrichtung mit einer optischen Signalisierungseinrichtung.
Fig. 10 zeigt eine Inhalationseinrichtung mit einer optischen und akustischen Signalisierungseinrichtung.
Fig. 11 a und 11 b zeigen zusammen ein Inhalationseinrichtungs-Set.
Fig. 12a und 12b zeigen eine weitere Inhalationseinrichtung 10, die sich durch einen sehr einfachen Aufbau auszeichnet.
Fig. 13a und 13b zeigen eine Ergänzung der Inhalationseinrichtung der Fig. 12a und 12b in Form eines ankoppelbaren Mundstücks sowie einer ankoppelbaren Inhalationseinrichtung.

### DETAILLIERTE BESCHREIBUNG DER AUSFÜHRUNGSBEISPIELE

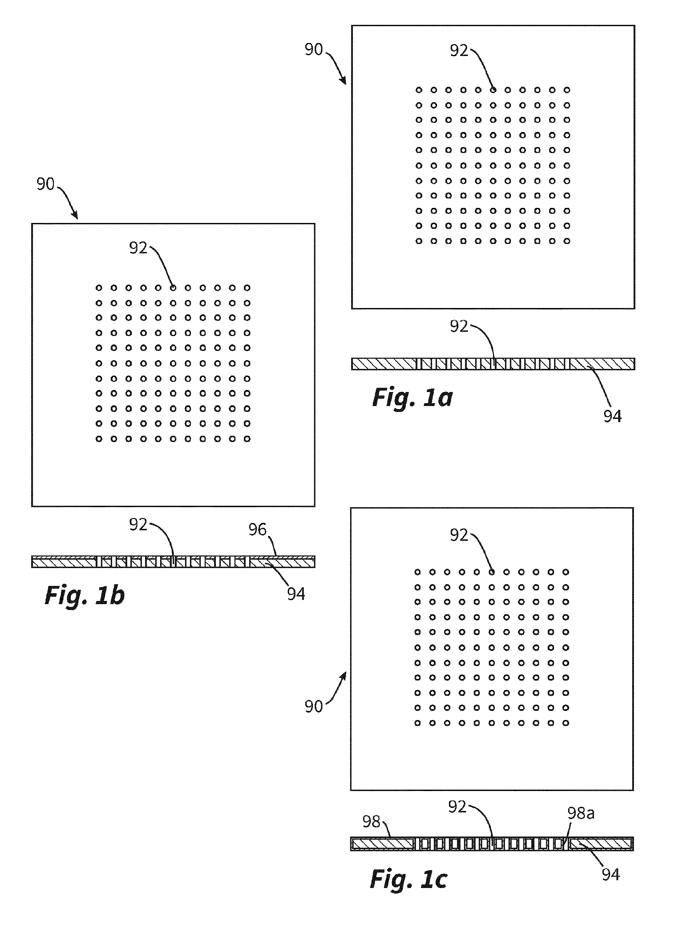

Die Fig. 1a bis 1c zeigen drei Düsenplatten 90 mit jeweils 121 Düsenöffnungen 92. Die Düsenöffnungen weisen einen Durchmesser von etwa 25 µm auf.

Die dargestellten Düsenplatten 90 dienen insbesondere der Verwendung in einer erfindungsgemäßen Inhalationseinrichtung. Dies wird nachfolgend noch beschrieben.

Die Düsenplatte 90 der Fig. 1a ist eine besonders einfache Düsenplatte, die als einheitlichen Werkstoff Silizium aufweist. In dieses Silizium sind die Düsenöffnungen 92 in einem Matrixraster eingebracht. Alternativ könnte zum Zwecke einer antibakteriellen Wirkung die Düsenplatte 90 der Fig. 1a auch als Ganzes aus Silber oder beispielsweise einer Silberlegierung oder einem anderen antibakteriell wirkenden Material gefertigt sein.

Die Fig. 1b zeigt eine Gestaltung, bei der die Düsenplatte 90 eine Grundplatte 94 und eine Beschichtung 96 aufweist. Während die Grundplatte 94 beispielsweise wiederum aus Silizium gefertigt sein kann, ist die Beschichtung 96 aus Silber oder einer Silberlegierung gebildet. Hierdurch wird Bakterienwachstum wirksam vermieden. Während bei Fig. 1b diese Beschichtung 96 nur einseitig dargestellt ist, kann dennoch auch eine zweiseitig beschichtete Platte vorgesehen sein. Eine Besonderheit der Gestaltung gemäß Fig. 1b ist, dass die Düsenöffnungen 92 nach der Beschichtung eingebracht wurden, so dass die Düsenöffnungen 92 innenseitig nicht oder nur partiell von der Beschichtung überdeckt sind. Dies kann aus Gründen einfacherer Herstellung einen Vorteil darstellen.

Die Gestaltung der Fig. 1c unterscheidet sich diesbezüglich. Auch hier ist eine Grundplatte 94 vorgesehen, die von einer Beschichtung 98 überdeckt wird. Da diese Beschichtung 98 jedoch nach Herstellen der Düsenöffnungen 92 aufgebracht wurde, überdeckt sie mit Abschnitten 98a auch die Innenseite der Düsenöffnungen 92.

Der partielle oder vollständige Überzug aus Silber oder einer Silberlegierung wirkt antibakteriell. Ist die Düsenplatte 90 auf der stromabwärtigen Seite beschichtet, so wirkt dies insbesondere einem Bakterienwachstum entgegen, das in dem niedergeschlagenen Flüssigkeitsfilm auf dieser Seite entstehen kann. Eine entsprechende Beschichtung auf der stromaufwärtigen Seite wirkt insbesondere auf die Flüssigkeit, die gegen Ende eines Austragvorgangs noch vor den Düsenöffnungen 92 steht. Dort ist eine antibakterielle Wirkung insbesondere deshalb zweckmäßig, da eine Verdunstung der dortigen Flüssigkeit durch die Düsenöffnungen 92 nur sehr langsam vonstattengeht und die Gefahr des Bakterienwachstums daher dort entsprechend groß ist.

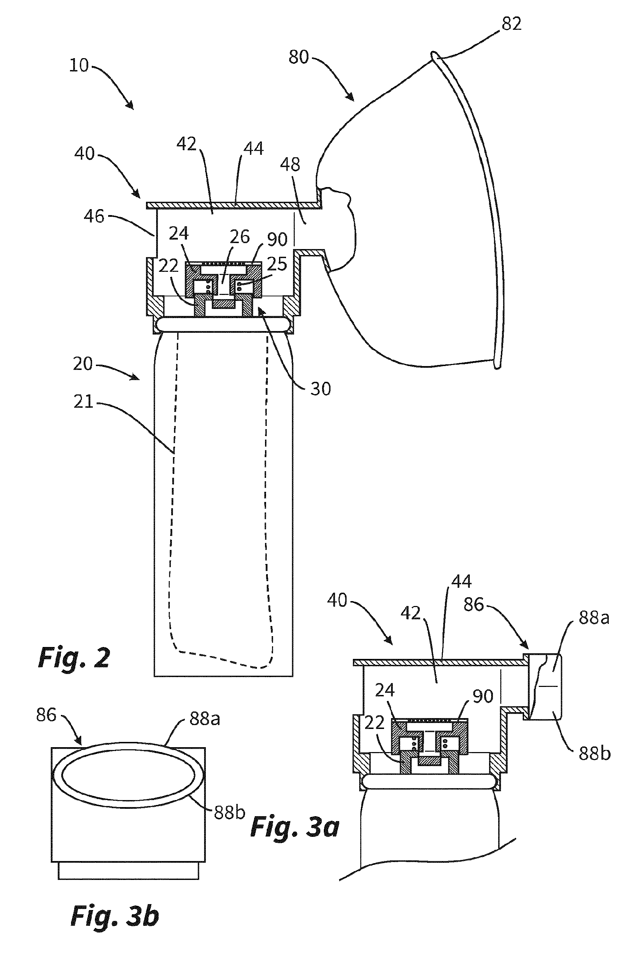

Die Fig. 2 sowie 3a und 3b zeigen sehr einfache Inhalationseinrichtungen 10 unter Verwendung solcher Düsenplatten 90. Die in den weiteren Figuren dargestellten Inhalationseinrichtungen bauen auf der grundsätzlichen Funktionalität dieser einfachen Inhalationseinrichtungen auf.

Die Inhalationseinrichtung 10 der Fig. 2 verfügt über ein als Druckbehälter ausgestaltetes zylindrisches Gehäuse 20, in dem eine Salzlösung gelagert ist. Diese Salzlösung kann mittels Treibgas druckbeaufschlagt sein. Eine Alternative hierzu liegt darin, dass der Druckbehälter mit Luft unter Überdruck befüllt ist und einen beutelförmigen Flüssigkeitsspeicher 21 aufweist, der in Fig. 2 exemplarisch gestrichelt dargestellt ist. Der Überdruck versucht, die Flüssigkeit aus dem Flüssigkeitsspeicher 21 in Richtung eines Austragkanals 26 und somit zur Düsenplatte 90 zu fördern. Zwischen der Düsenplatte 90 und dem Flüssigkeitsspeicher 21 ist ein Ventil 30 vorgesehen, welches gemeinsam durch einen zum Gehäuse 20 ortsfesten Stutzen 22 und einen demgegenüber in Hochrichtung verschieblichen Schieber 24 gebildet wird. Wie sich aus der Darstellung der Fig. 2 ergibt, muss der Schieber gegen die Kraft einer nicht dargestellten Feder niedergedrückt werden, damit der Austragkanal 26 geöffnet wird. Sobald dies der Fall ist, kann Flüssigkeit durch den Austragkanal 26 bis zur Düsenplatte 90 strömen. Unter dem sich dort dann einstellenden weitgehend konstanten Druck wird diese Flüssigkeit durch die Düsenöffnungen 92 der Düsenplatte 90 gedrückt und erzeugt so einen Inhalationsnebel.

Auf das Gehäuse 20 ist ein Applikatorkopf 40 aufgesetzt. Dieser umgibt einen Zerstäubungsraum 42, in den die durch die Düsenplatte 90 hindurch getretene Flüssigkeit in Nebelform gelangt. Das Gehäuse 44 des Applikatorkopfes 40 weist einen Lufteinlass 46 auf. Gegenüberliegend hierzu ist ein Luftauslass 48 vorgesehen, an den sich ein Applikationsstück anschließt. Das Applikationsstück in Fig. 2 ist eine Inhalationsmaske 80. Diese wird bestimmungsgemäß so am Gesicht des Benutzers angelegt, dass sie den Mund und die Nase vollständig überdeckt. Jeglicher Atemvorgang führt daher dazu, dass Luft durch den Applikatorkopf 40 hindurch angesogen wird, wo diese mittels der beschriebenen Vernebelung zur Inhalation vorbereitet wird.

Die Düsenplatte 90 kann wie auch bei den folgenden Ausführungsbeispielen in Art der Gestaltungen der Fig. 1a bis 1c ausgebildet sein. Eine nach außen weisende antibakterielle Schicht kann Bakterienwachstum verhindern, welches sich im Niederschlag des Inhalationsnebels auf der Düsenplatte 90 bilden kann. Eine zum Kanal 26 hin weisende Beschichtung sorgt dafür, dass in der Flüssigkeitsmenge, die innenseitig an der Düsenplatte 90 anliegt, kein Bakterienwachstum möglich ist. Dies ist besonders hilfreich, da die jenseits des Ventils 30 befindliche Flüssigkeit durch die Düsenöffnungen nur schwer verdunsten kann, so dass hier die Gefahr der Kontamination vergleichsweise hoch ist.

Die Fig. 3a und 3b zeigen die neben der Inhalationsmaske zweite Möglichkeit eines erfindungsgemäß zu nutzenden Applikatorstücks. Hierbei handelt es sich um ein Mundstück 86, welches entsprechend der Formgebung des menschlichen Mundes eine längliche Formgebung aufweist. Das Mundstück 86 ist an der Oberseite und der Unterseite mit Anlageflächen 88a, 88b versehen, die dem Anlegen der Lippen dienen und im Zusammenhang mit einigen der nachfolgend beschriebenen Ventileinrichtungen zweckmäßig sind, um hier ein Stützmoment einzukoppeln.

Bei den Ausgestaltungen der Fig. 2 sowie 3a und 3b ist die Art der Betätigung des Ventils nicht näher dargestellt. Das Niederdrücken des Schiebers könnte beispielsweise erfolgen, wenn dieser über einen nicht dargestellten, durch einen Schlitz in der Wandung des Gehäuses 44 geführten Betätigungshebel verfügt.

Besondere Ausgestaltungen des Ventils 30 bzw. der zugehörigen Öffnungsmechanik ergeben sich aus den folgenden Darstellungen.

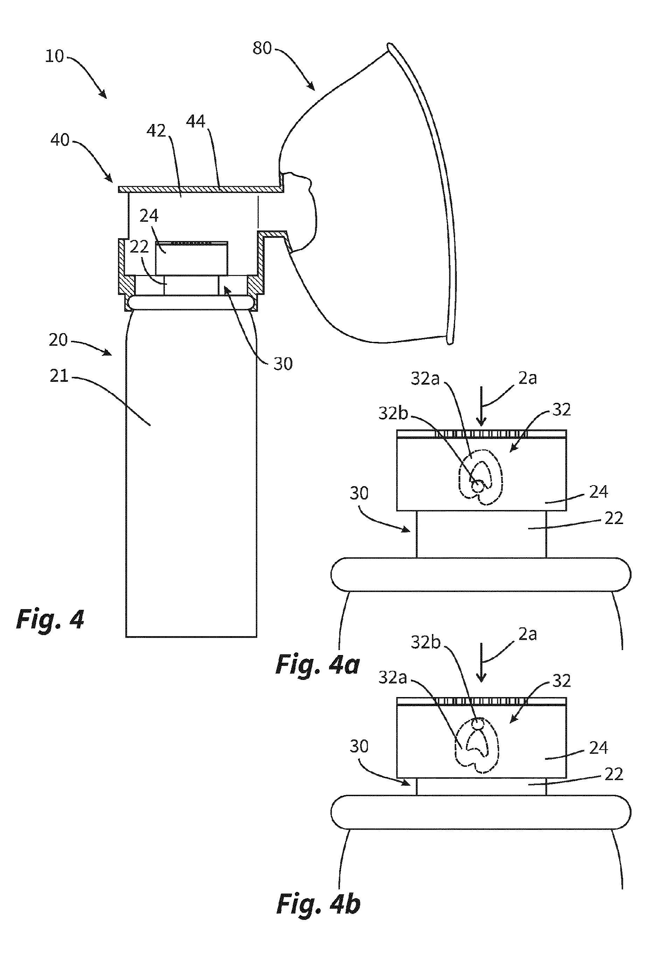

Die Fig. 4 sowie 4a und 4b zeigen ein Ausführungsbeispiel, bei dem der Stutzen 22 und der Schieber 24 so ausgestaltet sind, dass sie bistabil ein Schalten des Ventils 30 gestatten. Hierfür ist eine sogenannte Herzkurven-Mechanik 32 vorgesehen, die über eine gestrichelt dargestellte geschlossene Kulissenbahn 32a an der Innenseite des Schiebers 24 und einen hierein eingreifenden Nocken 32b an der Außenseite des Stutzens 22 verfügt. Der Nocken 32b kann an zwei Stellen, verdeutlicht in den Fig. 4a und 4b, eine stabile Position innerhalb der herzförmigen Kulissenbahn 32a einnehmen. Mit jeder Kraftbeaufschlagung des Schiebers 24 in Richtung des Pfeils 2a wird zwischen diesen beiden Positionen gewechselt. Hierdurch ist ein einfaches Umschalten zwischen einer Inhalationsnebelerzeugung und einem Ruhezustand möglich. Die Zugänglichkeit des Schiebers 24 von außen ist bei dieser Ausgestaltung nicht näher dargestellt. Es ist jedoch unschwer möglich, einen Fortsatz des Schiebers 24 durch eine Schlitzkulisse im Gehäuse 44 nach außen zu führen, so dass ein einfaches Umschalten möglich ist.

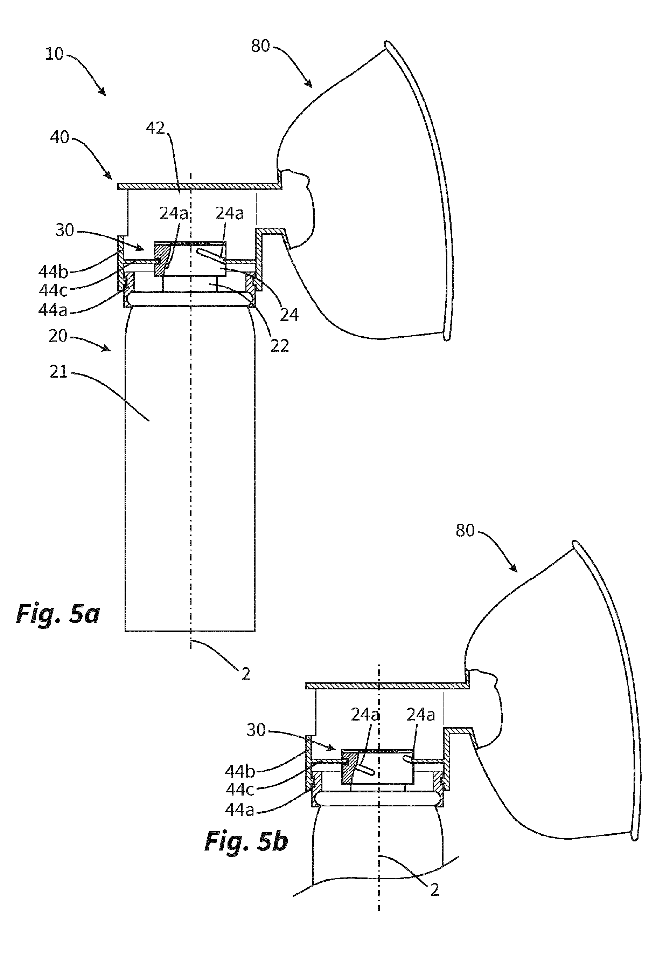

Bei der Ausgestaltung gemäß den Fig. 5a und 5b ist das Gehäuse des Applikatorkopfes 40 zweiteilig ausgebildet. Es weist einen unteren Abschnitt 44a auf, der verdrehgesichert am Gehäuse 20 angebracht ist. Demgegenüber ist um die Achse 2 ein oberer Abschnitt 44b drehbeweglich. Von diesem oberen Abschnitt 44b erstrecken sich zwei Ausleger 44c bis zum Schieber 24 und in am Schieber 24 vorgesehene schräggestellte Kulissennuten 24a. Da der Schieber 24 in nicht näher dargestellter Art und Weise drehfest zum Gehäuse 20 ausgestaltet ist, führt eine Drehung des oberen Gehäuseabschnitts 44b zusammen mit dem Applikationsstück 80 gegenüber dem Gehäuse 20 dazu, dass der Schieber 24 axial gegenüber dem Gehäuse 20 verschoben wird und somit ein Aktivieren und Deaktivieren der Inhalationsnebelerzeugung durch Öffnen und Schließen des Ventils 30 möglich ist. Von besonderem Vorteil ist, dass dieser Schaltvorgang einhändig erfolgen kann. Während der Benutzung der Inhalationseinrichtung 10 wird üblicherweise das Gehäuse 20 mit einer Hand umgriffen und hierdurch auch die Inhalationsmaske 80 gegen das Gesicht gedrückt. In dieser Stellung kann das Gehäuse 20 einfach als Ganzes und gemeinsam mit dem Schieber 24 gedreht werden, um die Erzeugung des Inhalationsnebels zu initiieren.

Diese einhändige Bedienung wird dadurch begünstigt, dass das Applikatorstück so ausgestaltet ist, dass es bei einer Dreh- oder Schwenkbewegung des Gehäuses 20 gegenüber dem Applikatorstück die Einkopplung eines Stützmomentes gestattet. Im Falle der Inhalationsmaske 80 wird dies durch eine umlaufende Dichtkante 82 erreicht, die am Gesicht anliegt und sich dort abstützen kann. Im Falle des Mundstücks 86 wird dies durch die für die Anlage der Lippen vorgesehenen Anlagefläche 88a, 88b erreicht. Durch leichtes Zusammendrücken der Lippen wird das Mundstück so fixiert, dass eine Relativbewegung des Gehäuses 20 zum Zwecke des Schaltens eines Ventils leicht möglich ist.

Fig. 5a zeigt die Inhalationseinrichtung 10 bei geschlossenem Austragventil 30. Die Fig. 5b zeigt den offenen Zustand.

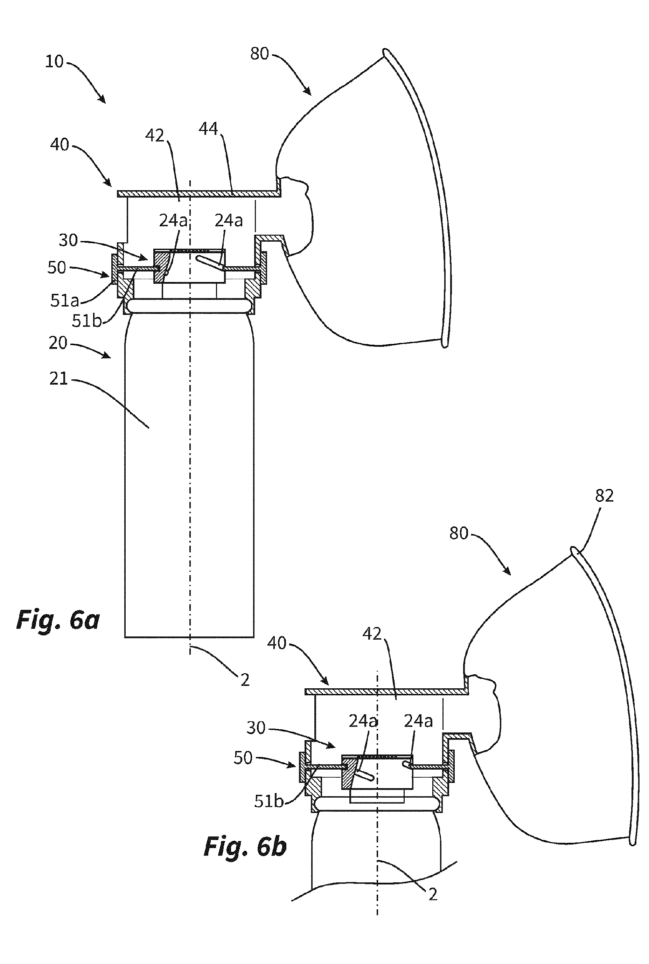

Die Gestaltung der Fig. 6a und 6b ist eine Variante zur Gestaltung der Fig. 5a und 5b. Das Gehäuse 44 des Applikatorkopfes 40 ist hierbei wiederum einstückig vorgesehen. Allerdings ist ein separates Schaltelement 50 am Applikatorkopf 40 angebracht, welches ebenfalls um die Drehachse 2 drehbar ist. Das Schaltelement 50 verfügt über einen von außen zugänglichen Schaltring 51 a und nach innen in die Kulissenspuren des Schiebers 24 ragende Ausleger 51 b. Durch Drehen des Schaltelements 50 über den Schaltring 51a kann das Ventil 30 geöffnet und geschlossen werden.

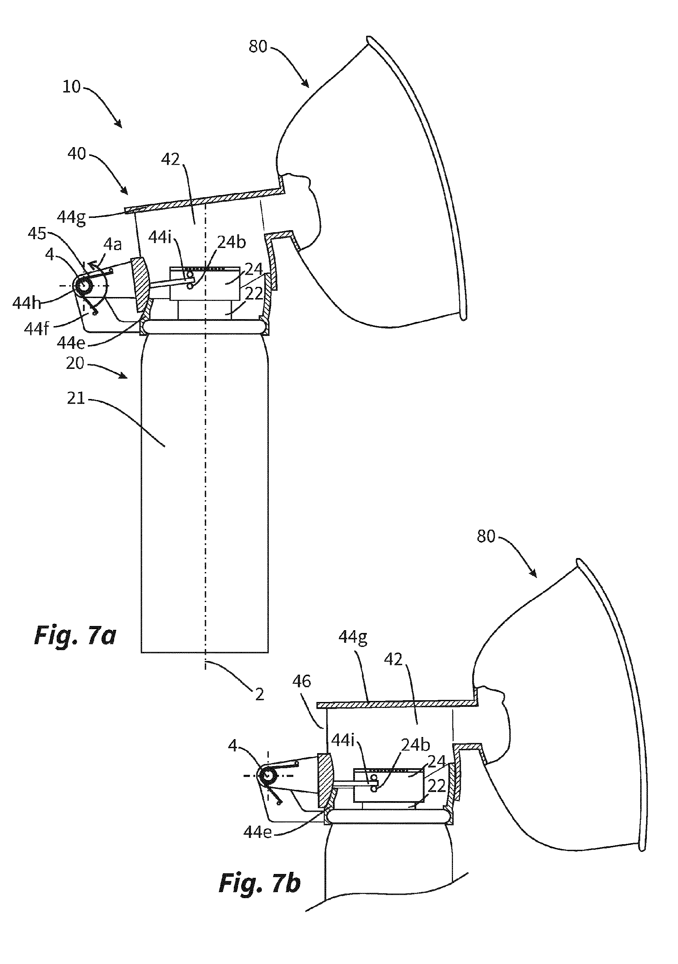

Bei der Gestaltung gemäß den Fig. 7a und 7b weist das Gehäuse 44 des Austragkopfes 40 wiederum eine Zweiteilung auf. Ein unterer Gehäuseabschnitt 44e ist fest am Gehäuse 20 angebracht. Ein oberer Gehäuseabschnitt 44g, an dem auch die Inhalationsmaske 80 vorgesehen ist, ist demgegenüber um eine Schwenkachse 4 schwenkbar. Zu diesem Zweck verfügen sowohl der untere Gehäuseabschnitt 44e als auch der obere Gehäuseabschnitt 44g über aneinander schwenkbar angelenkte Ausleger 44f, 44h, die gelenkig aneinander angebracht sind. Zwischen diesen Auslegern ist eine Schenkelfeder 45 vorgesehen, die den oberen Gehäuseabschnitt 44g gegenüber dem unteren Gehäuseabschnitt 44e in Richtung des Pfeils 4a momentenbeaufschlagt.

Zur Betätigung des Ventils 30 ist ein Ausleger 44i am oberen Gehäuseabschnitt 44g vorgesehen, der mit einem Nocken 24b am Schieber 24 zusammenwirkt. Wenn eine nur geringfügige Schwenkbewegung um einige Grad gegenüber der Ausgangsposition der Fig. 7a erfolgt, drückt der Ausleger 44b den Schieber 24 gegenüber dem Gehäuse 20 durch Kraftbeaufschlagung des Nocken 24b nach unten und bewirkt so das Öffnen des Ventils 30.

Wie auch bei den Ausgestaltungen der Fig. 5a und 5b ist hier eine sehr einfache einhändige Handhabung möglich. Wenn die Inhalationsmaske 80 gegen das Gesicht gedrückt wird, kann die Schwenkbewegung des Gehäuses 20 und damit das Öffnen und Schließen des Ventils 30 einhändig bewirkt werden.

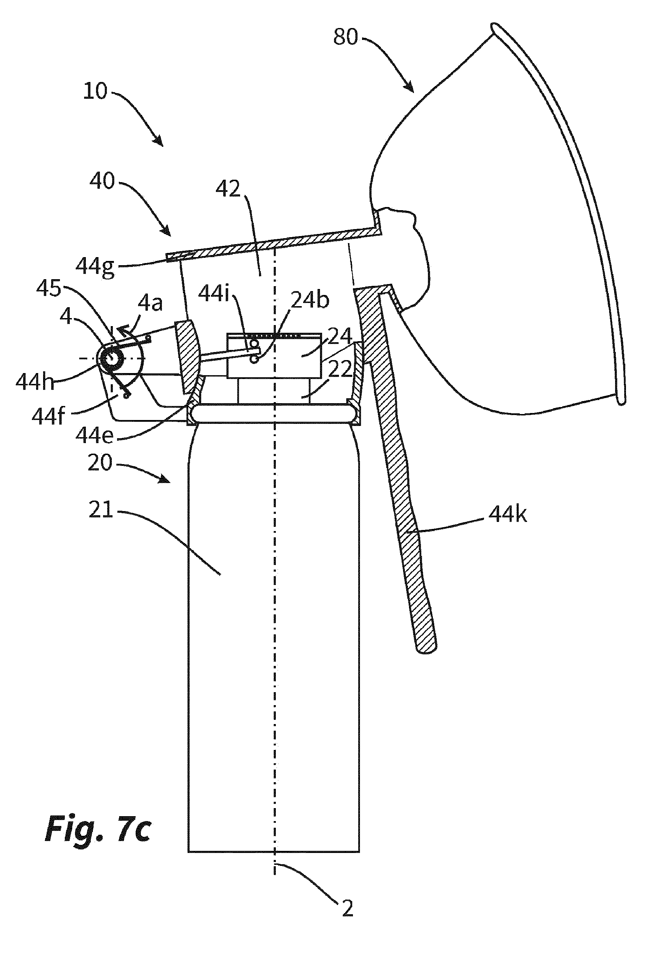

Fig. 7c zeigt eine ergänzte Gestaltung bei der zusätzlich zu den Komponenten der Ausführungsform der Fig. 7a und 7b ein Betätigungshebel 44k am Gehäuse des Applikatorkopfes 40 vorgesehen ist, Durch Umgreifen des Gehäuses 20 und des Betätigungshebels 44k mit einer Hand und Zusammenziehen der Hand kann ein Verschwenken und damit ein Öffnen des Ventils verursacht werden.

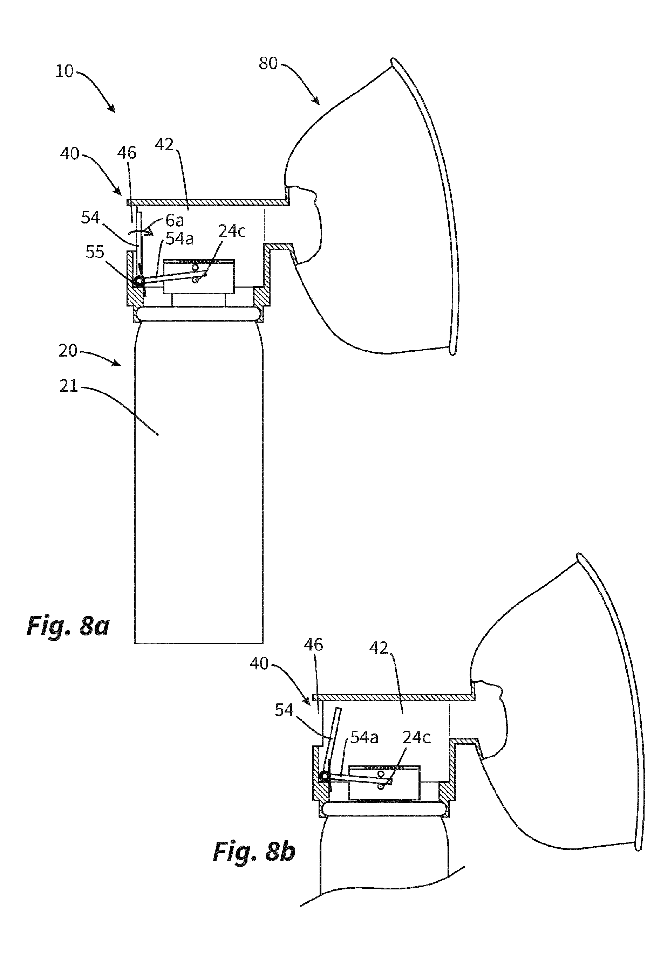

Die Fig. 8a und 8b zeigen eine Inhalationseinrichtung 10, deren Besonderheit darin liegt, dass durch Einatmen das Ventil 30 geöffnet wird. Hierfür ist dem Lufteinlass 46 eine Klappe 54 zugeordnet. Diese Klappe ist durch eine Federeinrichtung 55 in Richtung ihrer Stellung der Fig. 8a kraftbeaufschlagt. Fest mit der Klappe 54 verbunden ist ein Ausleger 54a, der wiederum auf einer Nocke 24c des Schiebers 24 aufliegt. Wird nun an der Inhalationsmaske 80 ein ausreichend großer Unterdruck erzeugt, so wird die Klappe 54 in Richtung des Pfeils 6a verschwenkt und somit der Schieber 24 niedergedrückt. Das Auslassventil 30 öffnet. Sobald kein ausreichender Unterdruck mehr vorliegt, kehrt die Klappe 54 unter Wirkung der Federeinrichtung 55 in die Ausgangsstellung der Fig. 8a zurück und das Ventil 30 schließt sich unter Wirkung der ventilintegrierten Feder wieder.

Die dargestellte Gestaltung ist sehr einfach gehalten. In der Praxis kann es von Vorteil sein, den Ausleger 54a nicht fest mit der Klappe 54 zu verbinden, sondern ein Übersetzungsgetriebe dazwischen vorzusehen, um über die Auslenkung der Klappe 54 eine größere Kraftwirkung zu erzielen.

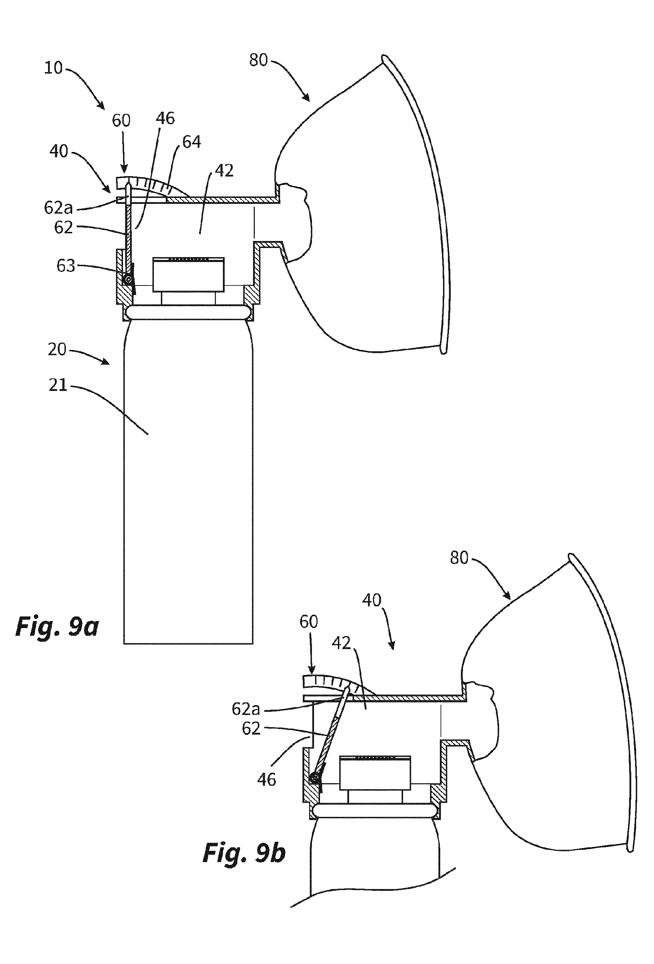

Ein ähnliches Konzept zu dem der Fig. 8a und 8b verfolgt die Ausgestaltung einer Inhalationseinrichtung 10 gemäß den Fig. 9a und 9b. Allerdings ist hier keine Steuerung des Auslassventils 30 vorgesehen. Dieses kann stattdessen auf einem anderen Wege, beispielsweise einem der beschriebenen Wege, geöffnet und geschlossen werden. Die bei der Ausgestaltung der Fig. 9a und 9b vorgesehene Klappe 62 am Lufteinlass 46, die durch die Feder 63 in Richtung ihrer Position der Fig. 9a gedrückt wird, trägt einen Signalabschnitt 62a in Form eines Pfeils, der in Abhängigkeit der Auslenkung der Klappe 62 eine Skala 64 entlangwandert. Somit lässt sich erkennen, wie stark der Benutzer inhaliert.

Eine solche Gestaltung ist insbesondere für die Verwendung der Inhalationseinrichtung durch Kinder hilfreich. Der Pfeil 62a und die Skala 64 gestatten es den Eltern, zu beurteilen, ob die Inhalation richtig erfolgt ist.

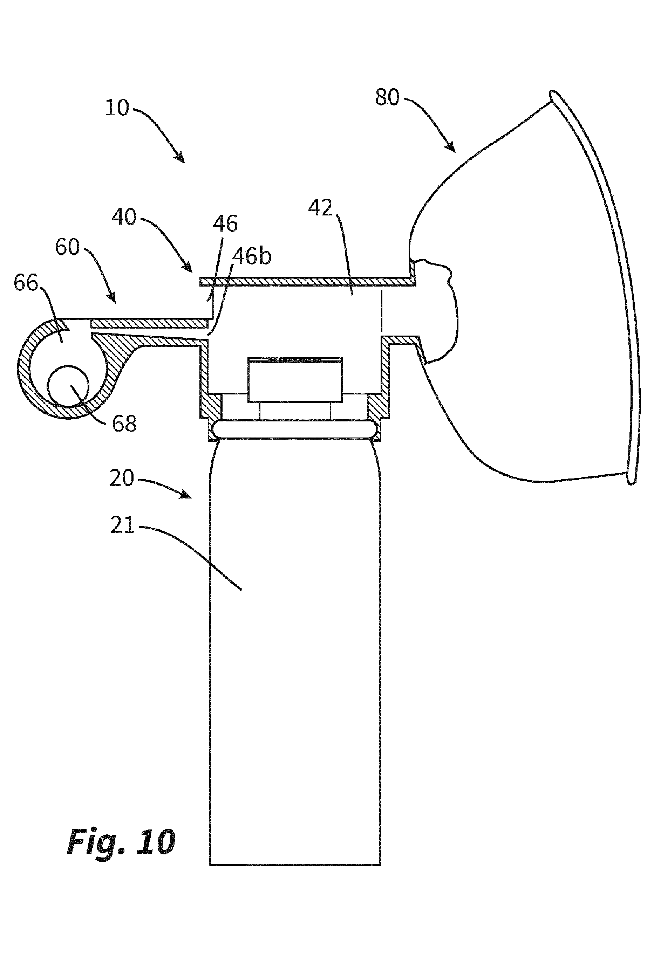

Die Ausgestaltung der Fig. 10 weist ebenfalls eine Signalisierungseinrichtung 60 auf. Diese ist in Art einer Trillerpfeife gestaltet. Hierfür ist der Lufteinlass 46 zweigeteilt. Ein Nebeneinlass 46b führt in einen Trillerpfeifenraum 66, in dem die für Trillerpfeifen charakteristische Kugel 68 angeordnet ist. Wird nun durch den Benutzer Luft eingeatmet, so stammt diese teilweise aus diesem Nebeneinlass 46b. Der zugrunde liegende Luftstrom ist in der Lage, ein Pfeifgeräusch zu erzeugen. Durch Verwendung transparenter Wandungen ließe sich auch ohne ein möglicherweise störendes pfeifendes Geräusch allein auf visuellem Wege erkennen, ob Luft durch den Nebeneinlass 46b eingeatmet wird und die Kugel in Konsequenz dessen rotiert.

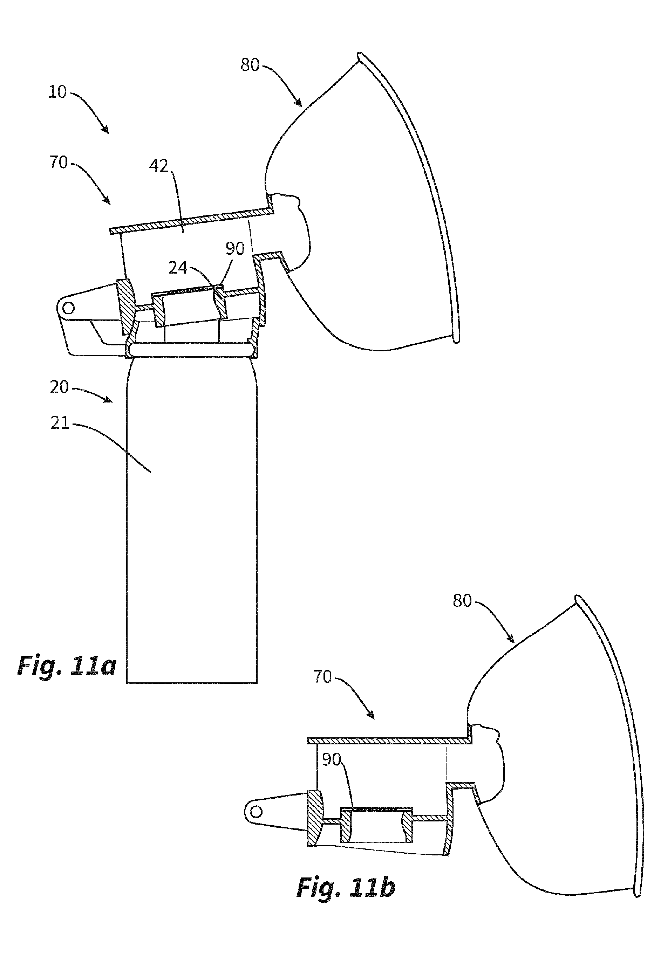

Fig. 11a zeigt eine Inhalationseinrichtung 10, die derjenigen der Fig. 7a und 7b ähnelt. Die Besonderheit ist hier, dass der Schieber 24 in den oberen Gehäuseabschnitt integriert ist. Das Ventil sollte dabei so beschaffen sein, dass es bei Abnahme des Schiebers 24 schließt.

Es ergibt sich eine zusammenhängende Baueinheit 70, die separat in Fig. 11b dargestellt ist und die ein einfaches Reinigen gestattet.

Ein erfindungsgemäßes Inhalationseinrichtungsset umfasst mehrere dieser Baueinheiten 70, so dass diese aus hygienischen Gründen getauscht werden können.

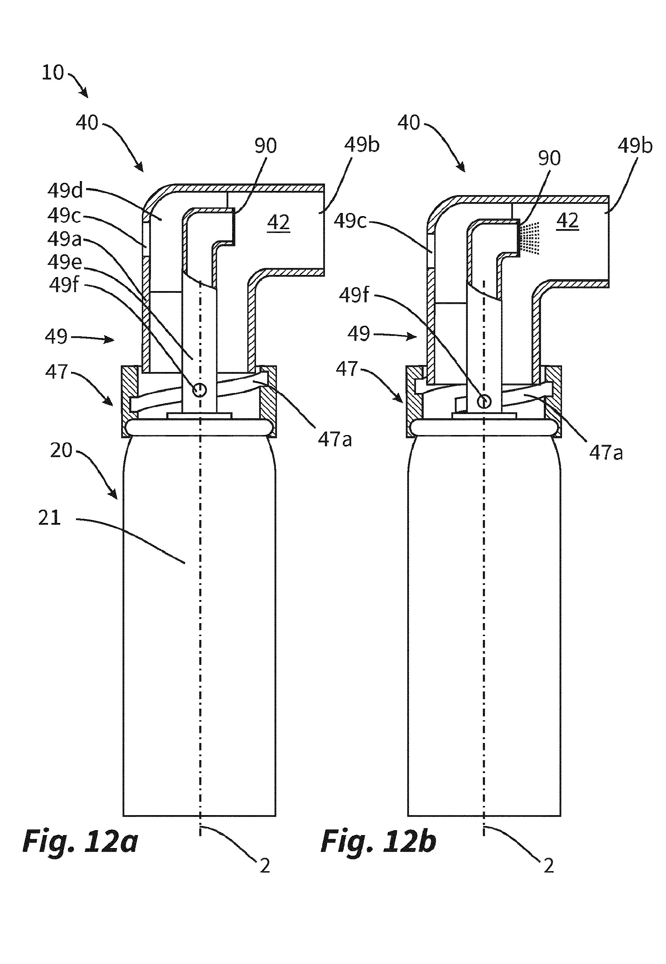

Fig. 12a und 12b zeigen eine weitere Inhalationseinrichtung 10. Bei dieser ist ein Applikatorkopf 40 aus sehr wenigen Bauteilen aufgebaut und daher günstig in der Herstellung.

Der Applikatorkopf verfügt über eine Basis 47, die drehfest mit dem Gehäuse 20 verbunden ist. Die Basis weist die Form eines Rings auf, wobei an der Innenseite einander gegenüberliegend zwei wendelabschnittsförmige Kulissenspuren 47a vorgesehen sind.

In die Basis 47 eingesetzt ist ein Auslassteil 49. Dieses verfügt über ein gekrümmtes Außenrohr 49a, welches in einer als Mundstück verwendbaren Öffnung 49b endet. Das Außenrohr 49a verfügt über eine Einströmöffnung 49c auf der der Öffnung 49b gegenüberliegenden Seite.

Im Außenrohr 49a ortsfest und vorzugsweise einstückig mit dem Außenrohr 49a verbunden ist ein Innenrohr 49e, welches vorliegend über eine Rippe 49d mit dem Außenrohr 49a verbunden ist. Das Innenrohr endet in einer Düsenplatte 90. Flüssigkeit, die durch das Innenrohr zur Düsenplatte 90 gefördert wird, wird dort in die Zerstäubungskammer 42 ausgetragen. Der Benutzer kann die zerstäubte Flüssigkeit durch Saugen an der Öffnung 49b inhalieren.

Zum Steuern der Zerstäubung ist das Auslassteil 49 gegenüber der Basis 47 um die Achse 2 drehbar. Am Auslassteil sind Führungsbolzen 49f vorgesehen, die sich vorliegend von einer Außenseite des Innenrohrs 49e nach außen bis in die Kulissenspuren 47a erstrecken. Eine Drehbewegung des Auslassteils 49 gegenüber der Basis 47 bewirkt daher auch eine axiale Relativverlagerung in Richtung der Achse 2. Dies verursacht das Öffnen und Schließen eines Ventils, welches in nicht dargestellter Weise innerhalb des Gehäuses 20 vorgesehen ist.

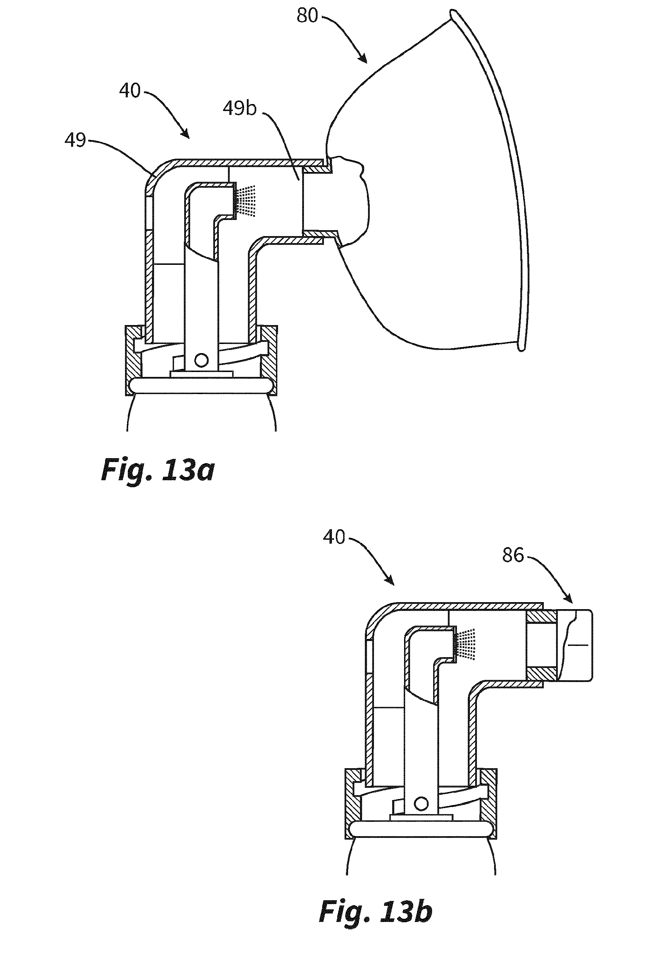

Das Ende des Außenrohrs 49 mit der Öffnung 49b kann unmittelbar als Mundstück verwendet werden. Die Fig. 13a und 13b verdeutlichen jedoch eine weitere Möglichkeit. So kann einer Inhalationseinrichtung mit einem Applikatorkopf entsprechend der Fig. 12a und 12b auch ein separates Mundstück 86 oder eine Inhalationsmaske 80 beigelegt sein. Diese umfassen jeweils einen Steckabschnitt, der in die Öffnung 49b eingeschoben werden kann und dort formschlüssig oder kraftschlüssig gehalten wird. So kann die Inhalationseinrichtung an individuellen Bedarf angepasst werden.

## Patentansprüche

1. Inhalationseinrichtung (10) zum Inhalieren einer Flüssigkeit in vernebelter Form mit den folgenden Merkmalen:
a. die Inhalationseinrichtung (10) verfügt über ein Gehäuse (20), welches einen Flüssigkeitsspeicher (21) umgibt, in dem die Flüssigkeit vor dem Austrag gelagert ist, und
b. die Inhalationseinrichtung (10) verfügt über einen Applikatorkopf (40) mit einem Zerstäubungsraum (42) und einem hiermit verbundenen Applikatorstück (80; 86), wobei das Applikatorstück (80; 86) entweder als Mundstück (86) zur Aufnahme im Mund eines Patienten oder als Inhalationsmaske (80) zur dichten Überdeckung des Mundes, der Nase oder des Mundes und der Nase oder als Adapterstück zur Anbringung eines Mundstücks (80) oder einer Inhalationsmaske (86) ausgebildet ist, und
c. die Inhalationseinrichtung (10) verfügt über einen Austragkanal (26), der den Flüssigkeitsspeicher (21) mit dem Applikatorkopf (40) verbindet,
**gekennzeichnet durch** das Merkmal:
d. die Inhalationseinrichtung (10) verfügt am Ende des Austragkanals (26) über eine Düsenplatte (90) mit einer Vielzahl von Düsenöffnungen (92) zur Erzeugung eines Inhalationsnebels, **durch** die hindurch die Flüssigkeit vom Flüssigkeitsspeicher (21) in den Zerstäubungsraum (42) geleitet wird.

2. Inhalationseinrichtung (10) nach Anspruch 1 mit den Merkmalen:
a. die Inhalationseinrichtung (10) verfügt über ein schaltbares Dreh-Auslassventil (30), durch das der Austragkanal (26) geöffnet und geschlossen werden kann, und
b. das Dreh-Auslassventil (30) ist durch eine Drehbewegung des Gehäuses (20) oder eines am Gehäuse oder am Applikatorkopf (80) vorgesehenen Schaltelements (50) gegenüber dem Applikatorstück (80; 86) schaltbar,
vorzugsweise mit den folgenden weiteren Merkmalen:
c. das Gehäuse (20) ist zumindest abschnittsweise als rotationssymmetrischer Körper ausgestaltet, dessen Mittelachse eine Haupterstreckungsachse (2) des Gehäuses (20) definiert, und
d. das Gehäuse (20) oder das Schaltelement (50) ist um die Haupterstreckungsachse (2) gegenüber dem Applikatorstück (80; 86) drehbar.

3. Inhalationseinrichtung (10) nach Anspruch 1 mit den Merkmalen:
a. die Inhalationseinrichtung (10) verfügt über ein schaltbares SchwenkAuslassventil (30), durch das der Austragkanal (26) geöffnet und geschlossen werden kann, und
b. das Schwenk-Auslassventil (30) ist durch eine Schwenkbewegung des Applikatorstücks (80; 86) gegenüber dem Gehäuse (20) schaltbar,
vorzugsweise mit den folgenden weiteren Merkmalen:
c. das Gehäuse (20) ist zumindest abschnittsweise als rotationssymmetrischer Körper ausgestaltet, dessen Mittelachse eine Haupterstreckungsachse (2) des Gehäuses (20) definiert, und
d. das Applikatorstück (80; 86) ist um eine zur Haupterstreckungsachse (2) orthogonale Schwenkachse (4) gegenüber dem Gehäuse (20) schwenkbar.

4. Inhalationseinrichtung (10) nach einem der Ansprüche 2 oder 3 mit dem Merkmal:
a. dem Auslassventil (30) ist eine Federeinrichtung (25, 45, 55) zugeordnet, die derart ausgebildet ist, dass ihre Federkraft stets versucht, das Auslassventil (30) zu schließen.

5. Inhalationseinrichtung (10) nach einem der vorstehenden Ansprüche mit den folgenden Merkmalen:
a. die Inhalationseinrichtung (10) verfügt über ein druckabhängig schaltbaren Auslassventil (30), durch das der Austragkanal (26) geöffnet und geschlossen werden kann, und
b. das druckabhängig schaltbare Auslassventil (30) ist als atembetätigtes Auslassventil ausgestaltet, welches durch Erzeugung eines Unterdrucks am Applikatorstück (80; 86) geöffnet werden kann und welches durch Erzeugung eines Überdrucks am Applikatorstücks (80; 86) oder durch Wegfall des Unterdrucks geschlossen werden kann.

6. Inhalationseinrichtung (10) nach einem der vorstehenden Ansprüche mit dem folgenden Merkmal:
a. die Inhalationseinrichtung (10) verfügt über eine bistabile schaltbare Auslassventileinrichtung (30),
vorzugsweise mit dem folgenden weiteren Merkmal:
b. die bistabile schaltbare Auslassventileinrichtung (30) ist mit einer Ventilmechanik (32) versehen, durch die Kraftbeaufschlagungen in gleicher Richtung (2a) alternierend das Öffnen und das Schließen der Auslassventileinrichtung (30) verursachen.

7. Inhalationseinrichtung (10) nach einem der vorstehenden Ansprüche mit dem Merkmal:
a. die Inhalationseinrichtung (10) verfügt über eine Signalisierungseinrichtung (60), die bei erfolgender Inhalation am Applikatorstück (80; 86) und durch den hierdurch erzeugen Unterdruck aktiviert wird,
vorzugsweise mit einem der folgenden weiteren Merkmale:
b. die Signalisierungseinrichtung (60) ist als Tonerzeuger ausgebildet, insbesondere als Pfeife oder als Tonerzeuger mit einem durch einen Luftstrom rotierenden Tongeber, oder
c. die Signalisierungseinrichtung (60) ist als optische Signalisierungseinrichtung ausgebildet und weist einen von außen sichtbaren Signalabschnitt (62a; 68) auf, der durch Unterdruck beim Inhalieren aus einer nicht signalisierenden Lage in eine Signalisierungslage gedrückt wird oder in Bewegung versetzt wird.

8. Inhalationseinrichtung (10) nach einem der vorstehenden Ansprüche mit den Merkmalen:
a. der Applikatorkopf (40) verfügt über ein drehfest mit dem Gehäuse verbundenes Basisteil (47) und über demgegenüber drehbares Auslassteil (49), welches den Zerstäubungsraum (42) umfasst, und
b. das Basisteil (47) und das Auslassteil (49) sind aneinander derart geführt, dass eine Drehbewegung des Auslassteils (49) gegenüber dem Basisteil (47) eine axiale Verlagerung der Auslassteils (49) gegenüber dem Basisteil (47) bewirkt, durch die ein Auslassventil der Inhalationseinrichtung (10) schaltbar ist.

9. Inhalationseinrichtung (10) nach einem der vorstehenden Ansprüche mit mindestens einem der folgenden Merkmale:
a. im Flüssigkeitsspeicher (21) ist Salzwasser enthalten, oder
b. im Flüssigkeitsspeicher (21) ist eine Flüssigkeit umfassend Wasser und mindestens einen der Zusätze aus der Gruppe umfassen Zimtöl, Teebaumöl, Salbeiöl, Thymianöl, Zitronenmelissenöl enthalten, oder
c. die Flüssigkeit im Flüssigkeitsspeicher (21) steht durch Beaufschlagung mit Treibgas, mit Druckluft oder durch eine vorgespannte Federeinrichtung unter Druck, oder
d. der Austragkanal (26) ist zumindest abschnittsweise mit einem antibakteriell wirkenden Material versehen, insbesondere mit Silber oder einer Silberlegierung, oder
e. das Mundstück (86) weist einen Auslass auf, dessen Breite größer ist als seine Höhe und der an der Oberseite und an der Unterseite über Anlageflächen (88a, 88b) zum Anlegen der Oberlippe und der Unterlippe verfügt, oder
f. die Inhalationsmaske (80) weist eine umlaufende Dichtkante (82) zur Anlage am Gesicht eines Benutzers auf, wobei die Dichtkante (82) so bemessen ist, dass sie den Mund oder die Nase oder den Mund und die Nase umschließen kann, oder
g. die Düsenplatte (90) umfasst Düsenöffnungen (92) mit einem Durchmesser zwischen 1 µm und 100 µm, insbesondere zwischen 2 µm und 10 µm, oder
h. die Düsenplatte (90) umfasst mindestens 9 Düsenöffnungen (92), vorzugsweise mindestens 16 Düsenöffnungen (92) und insbesondere vorzugsweise mindestens 25 Düsenöffnungen (92), oder
i. das Applikatorstück ist als Adapterstück zur Anbringung eines Mundstücks (80) oder einer Inhalationsmaske (86) ausgebildet und die Inhalationseinrichtung (10) verfügt weiterhin über ein Mundstück (86) zur Aufnahme im Mund eines Patienten oder eine Inhalationsmaske (80) zur dichten Überdeckung des Mundes, der Nase oder des Mundes und der Nase, oder
j. die Inhalationseinrichtung (10) verfügt über eine Betätigungshandhabe (44k), die gegenüber dem Gehäuse (20) verlagerbar, vorzugsweise verschwenkbar, ist, um hierdurch ein Auslassventil der Inhalationseinrichtung (10) zu öffnen.

10. Inhalationseinrichtung (10) nach einem der vorstehenden Ansprüche mit dem Merkmal:
a. das Applikatorstück (80; 86) und die Düsenplatte (90) sind Teil einer gemeinsamen Baueinheit (70), die werkzeuglos vom Gehäuse (20) lösbar ist.

11. Inhalationseinrichtungs-Set mit den Merkmalen:
a. das Set umfasst eine Inhalationseinrichtung (10) nach Anspruch 10,
b. das Set umfasst mindestens zwei Baueinheiten (70), die jeweils ein Applikatorstück (80; 86) und eine Düsenplatte (90) umfassen.

12. Düsenplatte (90), insbesondere für eine Inhalationseinrichtung (10) nach einem der vorstehenden Ansprüche, mit den folgenden Merkmalen:
a. die Düsenplatte (90) umfasst eine Vielzahl von Düsenöffnungen (92),
**gekennzeichnet durch** das Merkmal:
b. die Düsenplatte (90) ist zumindest abschnittsweise aus Silber oder aus einem Silber beinhaltenden Material gebildet.

13. Düsenplatte (90) nach Anspruch 12 mit mindestens einem der folgenden Merkmale:
a. die Düsenplatte (90) umfasst Düsenöffnungen (92) mit einem Durchmesser zwischen 1 µm und 100 µm, insbesondere zwischen 2 µm und 10 µm, oder
b. die Düsenplatte (90) umfasst mindestens 9 Düsenöffnungen (92), vorzugsweise mindestens 16 Düsenöffnungen (92) und insbesondere vorzugsweise mindestens 25 Düsenöffnungen (92).

14. Düsenplatte (90) nach einem der Ansprüche 12 oder 13 mit den folgenden Merkmalen:
a. die Düsenplatte (90) weist eine Grundplatte (94) aus einem Material auf, welches kein Silber oder Silber beinhaltendes Material beinhaltet,
b. auf der Grundplatte (90) ist eine Schicht (96; 98) aus Silber oder aus einem Silber beinhaltenden Material vorgesehen,
vorzugsweise mit einem der folgenden weiteren Merkmale:
c. die Schicht (96; 98) aus Silber oder Silber oder aus einem Silber beinhaltenden Material ist nach Einbringung der Düsenöffnungen (92) durch Bedampfen oder Eintauchen auf die Grundplatte (94) aufgebracht, so dass die Düsenöffnungen (92) an ihren Innenseiten zumindest teilweise mit dieser Schicht bedeckt sind, oder
d. die Düsenöffnungen (92) sind nach Bedampfen oder Eintauchen der Grundplatte (94) zum Zwecke der Herstellung der Schicht aus Silber oder Silberoxid eingebracht, so dass die Düsenöffnungen (92) an ihrer Innenseite zumindest teilweise frei von dieser Schicht sind.
